# EUROPEAN PATENT SPECIFICATION

(11) **EP 2 510 931 B1**
(45) Date of publication and mention of the grant of the patent: **13.12.2017**
(21) Application number: 10823674.6
(22) Date of filing: 07.10.2010
(51) Int. Cl.: A61K 31/355, A61K 31/375, A61K 35/64, A61K 36/18, A61P 15/08

(54) **MEDICAMENT FOR PREVENTING AND TREATING ADENOMA OF THE PROSTATE GLAND AND PROSTATITIS**
MEDIKAMENT ZUR PRÄVENTION UND BEHANDLUNG VON ADENOMEN DER PROSTATA UND PROSTATITIS
MÉDICAMENT POUR LA PRÉVENTION ET LE TRAITEMENT DE L'ADÉNOME DE LA PROSTATE ET DE LA PROSTATITE

(30) Priority: 12.10.2009 RU 2009137793
(43) Date of publication of application: 17.10.2012
(73) Proprietor: Obshestvo S Ogranichennoj Otvetstvennostju "PARAFARM", Penza 440023 (RU)
(72) Inventor: ELISTRATOV, Dmitriy Gennadjevich, Penza 440060 (RU)
(74) Representative: Engel, Christoph Klaus
(86) International application number: PCT/RU2010/000562
(87) International publication number: WO 2011/046470

(56) References cited:
- WO-A1-2010/104418
- RU-C1- 2 066 963
- RU-C2- 2 207 137
- RU-C2- 2 225 213
- 'Vse o mede i produktakh pchelovodstva.' PYLTSA, [Online] 2005, pages 1 - 4 Retrieved from the Internet: <URL:http://honey-online.narod.ru//pollen and_perga html>

## Description

The invention relates to means for the prevention and treatment of adenoma of the prostate gland and prostatitis.

The best biostimulants for prevention and treatment of adenoma and prostatitis are pollen-based preparations. Known is the pollen and lactose based Tonus preparation approved by the General Board of Health under the President of the Russian Federation (TU-64-02-06-244-91) and available from Biocor company. Known is the Cernilton medicament available from Graminex LLC (US) (htpp://www.cernilton.ru/). Cernilton® has an anti-inflammatory, antiedematous action, stimulates erectile activity and improves sexual potency. Cernilton ® is made from the fermented plant pollen extract.

The prior art closest to the invention by its technical essence and the achieved effect is the Leveton natural poly-vitaminic complex (RU 2066963, 27.09.1996) comprising pollen, Leuzea, ascorbic acid, α-tocopherol, propolis, calcium stearate, talc and lactose at the following ratio (wt. %): pollen: 50-51, Leuzea: 10, ascorbic acid: 3.0-3.5, α-tocopherol acetate: 0.15, propolis: 2, calcium stearate: 0.92-0.95, talc: 3, lactose: balance.

However, Leveton has an essential disadvantage because Leuzea as one of its ingredients is contraindicated for hypertensive patients and since the problems with sexual potency, adenoma and prostatitis are most often experienced by elderly people who also suffer from hypertension, the number of patients able to use Leveton is significantly limited.

It is an object of the present invention to enhance the positive therapeutic action without showings of a side effect.
The above object is achieved as follows.

A medicament for prevention and treatment of adenoma of the prostate gland and prostatitis is provided, comprising pollen, ascorbic acid, vitamin E, drone brood and excipients at the following ratio (wt. %):
drone brood: 2-20
pollen: 5-30
ascorbic acid: 5-10
vitamin E: 1-5
excipients: balance.

The medicament for prevention and treatment of adenoma of the prostate gland and prostatitis may be shaped as a tablet.
Calcium stearate: 0.92-0.95, talc: 3-6 and lactose as balance may be used as excipients.
According to the invention, instead of Leuzea the drone brood is used which has no negative effect on the arterial tension and at the same time has a large number of enzymatic functional groups of sulfide groups as well as hormones such as testosteroids, progesterone and estradiol. Due to such combination of substances, the drone brood contributes to an accelerated recovery of biochemical and mass-metric characteristics of testicles and prostate thereby acting as a stimulant of the central mechanisms regulating the intensity of androgens formation.
The drone brood has the following chemical composition: proteins: 10-20%; carbohydrates: 1-5.5%; fats: 5-6.3%; amino acids: 11.4%; glucose: 3.18-5%; fructose, saccharose up to 0.5%.

Trace elements (mg %): K: 0.50, Na: 38, Ca: 14, P: 189, Mg: 2, Fe: 3.23, Mn: 4.40, Zn: 5.54, Cu: 2, Cr, Co, Ni, Ag, Au, etc.

Vitamins (water- and liposoluble): A: 0.54 IU/g: xanthophyll: 0.297 mg %; β-carotin: 0.426 IU/g; B2: 0.739 mg %; D: 950 IU/g; choline: 442.8 mg %; nicotinic acid: 15.8 mg %.

In the modern sense of the term, adenoma of the prostate gland is a manifestation of climacterium virile accompanied by extinction of sexual activity.

The term "adenoma of the prostate gland" is rather conventional since it not the prostate gland itself that enlarges but small glands of the submucosal layer of the urinary bladder neck, which form three islets, namely, two lateral islets called a periurethral group, and one rear islet (towards the rectum) called pericervical group so that the diseases should be rather termed as the adenoma of external glands of prostata. The functions of external glands of prostate are not clear in full up to now. They are believed to be endocrine glands antagonistic to the male sexual glands and enlarge when atrophic processes occur in the prostate gland by the time of extinction of sexual activity. Therefore, it is important to maintain the level of body testosterone. The drone brood contains 10 times more steroid hormones than the pollen.

The drone brood contains the following hormones (nmole/100 g):
testosterone: 0.307
progesterone: 51.32
prolactin: 410.0
estradiol: 677.6.

Accordingly, the drone brood reduces the activity of inflammatory and autoimmune processes, prevents testosterone from transforming into dihydrotestosterone and controls the excessive growth of the prostate gland cells. The drone brood improves the quality of sperm by increasing the number of normal active sperm cell forms.

It has been found that the drone brood does not change the human hormonal status ((Krivtsov N.I. et al., Theory and Methods of Apitherapy, Moscow, 2007).

As a result of the search carried out in the sources of scientific-technical and patent literature, no composition has been found having a similar combination of essential features whereby the same positive effect is achieved. Therefore, the proposed invention offers a technical solution which is novel, has inventive step and is industrially applicable. The medicament is available as tablets. The lower limit is defined by the ease of use of the proposed complex, i.e., application of a less number of tablets. The upper limit is defined by the difficulty of shaping a tablet at a higher percentage ratio because it would fall to pieces.

### Example 1

An exemplary formulation of the complex for a 500 mg tablet:
drone brood
pollen
ascorbic acid
vitamin E
excipients

### Example 2

An exemplary formulation of the complex for a 500 mg tablet:
drone brood: 100 mg
pollen: 100 mg
ascorbic acid: 25 mg
vitamin E 20: mg
excipients: 255 mg.

The medicament has been tested in elderly volunteers, mainly in the group of hypertensive patients.

As a result of the conducted treatment, it may be concluded that said medicament is indicated for elderly people, namely, the inventive formulation is both qualitatively and quantitatively intended for prevention and treatment of adenoma of the prostate gland and prostatitis so that eventually potency is improved on a regular application thereof.

## Claims

1. A medicament for prevention and treatment of adenoma of the prostate gland and prostatitis, comprising pollen, ascorbic acid, vitamin E, excipients, **characterized in that** it further comprises drone brood at the following ratio of components (wt. %):
drone brood: 2-20
pollen: 5-30
ascorbic acid: 5-10
vitamin E: 1-5
excipients: balance.

2. The medicament according to claim 1, **characterized in that** it is provided as a tablet.

## Patentansprüche

1. Medikament zur Prävention und Behandlung von Adenomen der Prostata und Prostatitis, umfassend Pollen, Ascorbinsäure, Vitamin E, Arzneiträger, **dadurch gekennzeichnet, dass** es weiterhin Drohnenbrut mit folgendem Verhältnis der Bestandteile (Gewicht-%) umfasst:
Drohnenbrut: 2-20
Pollen: 5-30
Ascorbinsäure: 5-10
Vitamin E: 1-5
Arzneiträger: Rest

2. Medikament nach Anspruch 1, **dadurch gekennzeichnet, dass** es in Tablettenform bereitgestellt ist.

## Revendications

1. Médicament pour la prévention et le traitement de l'adénome du gland de la prostate et de la prostatite, comprenant du pollen, de l'acide ascorbique, de la vitamine E, des excipients, **caractérisé en ce qu'**il comprend en outre du couvain mâle avec la proportion suivante de composants (pourcentage en poids) :
couvain mâle : 2 à 20
pollen : 5 à 30
acide ascorbique : 5 à 10
vitamine E : 1 à 5
excipients : équilibrage.

2. Médicament selon la revendication 1, **caractérisée en ce qu'**il prévu sous forme de comprimé.
